# EUROPEAN PATENT SPECIFICATION

(11) **EP 0 996 423 B1**
(45) Date of publication and mention of the grant of the patent: **08.06.2011**
(21) Application number: 98912540.6
(22) Date of filing: 03.04.1998
(51) Int. Cl.: A61K 9/00, A61K 39/395, A23L 1/06

(54) **A METHOD FOR PRODUCING JELLY SWEETS WHICH CONTAIN ANTIBODIES**
VERFAHREN ZUR HERSTELLUNG VON ANTIKÖRPER ENTHALTENDEN GUMMIBÄRCHEN
PROCEDE DE PRODUCTION DE PRODUITS SUCRES CONTENANT DE LA GELEE

(30) Priority: 03.04.1997 WO PCT/FI97/00208
(43) Date of publication of application: 03.05.2000
(73) Proprietor: Hakalehto, Elias, 70110 Kuopio (FI); Oy Reagena Ltd, 70900 Toivala (FI)
(72) Inventor: Hakalehto, Elias, 70110 Kuopio (FI)
(74) Representative: Pitkänen, Hannu Alpo Antero
(86) International application number: PCT/FI1998/000298
(87) International publication number: WO 1998/043610

(56) References cited:
- WO-A-93/15736
- WO-A-94/21284
- DE-A- 4 324 859
- WPI/DERWENT'S ABSTRACT, No. 95-070233, week 9510; & JP,A,06 345 668 (SHIBAYAGI KK) 20 December 1994.

## Description

The object of the invention is a method producing jelly sweets which contain antibodies.

Nature has its own probiotic components like antibodies. Antibody proteins are found from most of the animals, and they are key components in the humoral immunity and providing a primary defence against the invading microbes. Antibodies consist of four polypeptide chains that conform the functional molecule, which has two antigen binding sites and a frame that has other biological functions. A binding specificity is a very important function of antibodies enabling them to be used in identification and elimination of foreign structures such as microbes. Ones the defence mechanism of body is activated by the foreign material, the specific antibodies are formed against it. The unique binding ability of antibodies helps body in eliminating the harmful material. In addition of natural immunity, the defence mechanisms of body can be further strengthened by delivering external antibodies that exert preventive and therapeutic effects. Ones the body encounters pathogenic microbes, the onslaught of disease may take place within a few days or even few hour from the invasion. Due to activated immune system, appearance of specific antibodies in the body fluids begins reaching its therapeutic level within few days or weeks leading to the lost of virulence and recovery of the disease. Declining of the antibody titer to normal takes a few weeks after the foreign material has been removed from the body.

The ability of antibodies to bind their target molecules very specifically has been exploited in biomedical research and in the diagnosis of diseases for several decades. In addition of research and diagnostics, therapeutic applications of antibodies have risen interests. These innovations are based on the use of experimentally manufactured antibodies that mimic function of natural antibodies in body. In preventive applications, the externally delivered antibodies may slow down the invasion of pathogenic microbes until the natural immunity is ready to take control over the invasion. The possible targets of antibody therapies vary from the classical use of horse immune serum against difficult snake bites to modern applications in controlling numerous immunological defects.

Despite of apparent advantages of using antibodies as therapeutic components, their widespread use has confronted many problems including high cost of their production and safety. The antibodies purified from serum of warm-blooded animals may contain adventitious infectious agents, also injection with allotypic antibodies may contribute serum sickness which in its worse may be lethal.

According to our present knowledge, hen egg yolk antibodies provide inexpensive and convenient alternative to mammalian antibodies in the applications in which the antibody is delivered to the gastrointestinal tract. Due to our effective purification methods, we have been able to use egg yolk antibodies successively in many diagnostic application. According to many publications, hen egg yolk antibodies prepared after experimental immunization with intact microbes or microbial antigens proved to be useful in prevention and recovery of some infectious diseases in animal models. The procedure of preparing hen egg yolk antibodies avoids bleeding, thus reducing stress of production animals. This makes it easier to fulfill the legal requirements set for experimental animals, and furthermore it reduces the risk of contaminations from blood.

Antibodies against several types of antigens have been produced in hens. These include native and synthetic proteins and polypeptides, intact and different structures of microbe cells. Hens have been found to be very effective producers of antibodies, the production equalling that of large domestic animals. Specific antibodies can be isolated from the egg yolk even after two weeks from the first immunization, after which the production continues for at least 100 days. One egg may contain as much as 150-200 mg of antibodies, from which up to 5% may be antigen specific. In terms of saved expenses per therapeutic dose, egg yolk technology beats any other procedure for the comparative applications.

The antigens originating from pathogenic microbes can be treated with chemicals or gamma irradiation for eradicating their virulency in man or other animals. Once the desired level of antibodies has been reached in the yolks, the yolks are homogenized and/or the antibodies are purified to a homogeneity, filtrated for sterilization, and added to the final formulations to give their probiotic and therapeutic effect.

Mucous membranes of respiratory tract provide a gateway to the body for many harmful microbes. Breathed air and eaten food bring along micro-organisms which attach to the mucous membranes of respiratory or GI tract, where they can penetrate to the body and cause infection and outbreak of a disease. The body defends itself against the invaders by secreting immunoglobulin -A and -E (IgA, IgE) to mucous membranes, where they bind to the harmful organisms preventing their attachment to the tissues, and thereby their penetration to the body. Sometimes this mechanism, and the others involved, fail to prevent invasion partially due to the lack-period of delivering reactive antibodies to the membranes. Sometimes this leads to the outbreak o fa disease. In the course of the disease, reactive antibodies are raised in blood where they inactivate the invader, while the mucosal antibodies prevent the invasion of new organisms. When the external antibodies are delivered to the mucous membranes, they may help the bodys' natural immune system by providing the first-line defence preventing the attachment and the penetration of invading micro-organism. Potential targets of the additional antibodies are:
- infections of gastrointestinal and respiratory tract
- dental caries
- otitis and other mid-ear infections

Dental caries is, according to the present knowledge, caused by bacteria in mouth. The experiments making use of specific antibodies have demonstrated the efficacy of those antibodies in prevention of dental caries in animal models.

Infections of respiratory tract with their secondary complications can cause severe diseases particularly in children and aged people. The treatments, if any available, of these diseases are ineffective and require long period of time. The cilial structures in the cavities of the upper respiratory tract transports particles to pharynx. This mechanism is an important part of the body's defence system. Infections in the respiratory tract destruct the cilial structure, exposing body to new invators. At its worse, this may lead to recurred diseases. Delivering of reactive antibodies against the pathogen concerned may prevent the actual infection or stop recurrence of new infections.

The purpose of the invention is to present a simple method for producing jelly sweets containing antibodies. Furthermore the purpose is to present a method with low production costs.

The purpose of the invention is achieved with the method described in the claims.

This invention uses general properties of antibodies to exert therapeutic effects, and a convenient way for delivering those antibodies to gastrointestinal (GI) and respiratory tract in a formulation based on a mixture of normal jelly sweets, such as marmalade, and homogeneous or unhomogenous antibodies. The antibodies in the mixture may be of egg yolk or colostrum origins. Colostrum is the first milk that a cow produces after calving, and is rich of antibodies. According to the invention, the principal benefits of using a jelly sweets as a medium for delivering antibodies are as follows:
- pH of the medium can be set around neutral that is optimal for antibodies
- polysaccharide composition of jelly sweets support stability of antibodies
- jelly sweets can harbor adequate amounts of antibodies without lost of their taste
- manufacture of jelly sweets occurs in low temperature before the sweets are allowed to cool without any raising of temperature afterwards.
- when chewed, sucked or bitten, jelly sweets dissolve slowly to the interior of mouth where they have time enough to occupy mucous membranes. Repeated doses of such a jelly sweets enhance their probiotic and therapeutic effects.

The antibodies used in the invention may be natural colostrum or egg yolk antibodies, or may be raised experimentally by immunization with the antigens capable of eliciting therapeutic response. The antibodies may be homogeneous or partially purified from the above mentioned sources. For purification, water extraction, salt precipitation or micro- and ultrafiltration methods separately or in combinations, are effective. The jelly sweets may be made by the general procedures, and the antibodies can be added in liquid or solid form at any stage of the production.

In general, antibodies can be produced against any known micro-organisms. Especially potential targets for the innovation are:
- influenza, parainfluenza, RSV-, adeno-, rhino-, EBV-, cytomegalo-, rota- and entero viruses
- bacteria or viruses that cause inflammations of respiratory or GI tract
- microbes that cause teeth and mouth diseases (e.g. *Streptococcus mutans*)
- other microbes that produce harmful substances in the body.

The antibodies can also be produced to eliminate influences of toxins and other substances that can be either toxic or harmful (e.g. environmental toxins). With the help of this invention it is possible to increase the rinsing of harmful organisms or compounds from body. It can also prevent the function of microbes or they reproduction by preventing transport of essential molecules through the cell wall, or by moving or binding the microbial cells causing their disturbtion. The invention may also aggregate harmful microbes in a way that they become rinsed from the body, or become delivered to the other places of body where they lose their virulency.

Antibodies can also be linked to other molecules to increase their health-promoting effects by using general coupling methods. These effector molecules can be antibiotics, obsonating compounds, lectins, components that inhibit or stimulate growth of certain microbes, enzymes, cofactors or other corresponding molecules. The binding of these components to the antibodies can be accomplished with different methods either chemically or physically.

In manufacture of the sweets containing bioactive antibodies, it is utmost important that the conditions during their production and storage are controlled to preserving bioactivity, since abnormal pH, temperature, salt or unsuitable chemical concentration may destroy bioactivity of antibodies. It is also possible to add buffered solutions, trehaloses or other molecules helping preservation of antibodies.

### Example 1.

Antibodies were produced in a chicken (A) by injecting it with a peptide consisting of amino-terminal peptide 1-29 of human parathyroid hormone. The antibodies produced against this peptide were isolated and purified from the yolk of the eggs laid by the chicken A with a previously known procedure.

25 mg of antibody produced was added to a marmalade candy (Vihreä kuula, Fazer, Helsinki, Finland) by removing corresponding amount of marmalade from inside the candy and joining the parts together again. The weight of the candy was 21.5 g, and pH was 3.51.

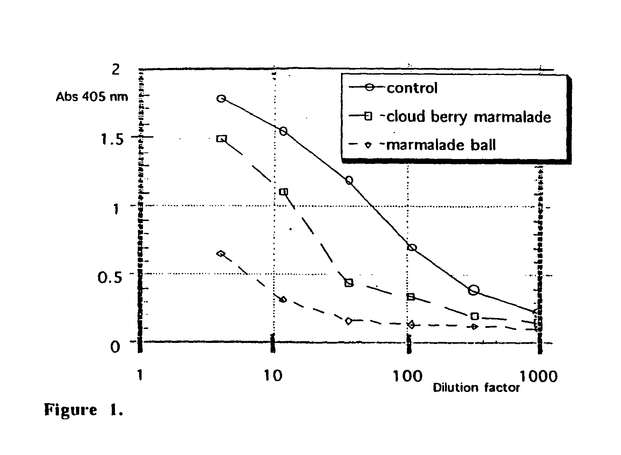

The marmalade candy containing the antibody was given to the human subject. After 5 min, 10 ml of sterile saline solution was given to the subject for rinsing the mouth for 30 seconds. This served as a sample that was examined as in the Fig. 1. Unsupplemented marmalade that was chewed 5 min before the experiment served as a blank.

The results are seen in the Fig. 1, which has been explained later.

### Example 2.

A candy containing therapeutic antibody was produced with this procedure: 200 ml of water was heated to boiling point and 5 g of agar was added. The solution was boiled 30 minutes to get the agar to solute. After that 14.5 g of sugar, 3.7 g of xylitol and 50 g of cloud berry jam (Kesko, Finland) was added. The solution was frozen to 55 °C and poured into 7 ml mold. After that, 25 mg of the antibody according to the example 1 in 0.4 ml saline was added. During the addition, the temperature was around 40 °C and the solution had started to mold. The pH of the final marmalade was 4.27.

The human subject ate the marmalade, and after that, a sample was taken and analysed as in the example 1. The unsupplemented marmalade prepared, chewed and tested accordingly served as a blank.

The results are seen in the Fig. 1.
Fig. 1 presents therapeutical marmalade experiments 1 and 2. The experiments were done as described in the text. After sampling, aliquots of samples were diluted with the Elisa assay buffer (PBS-2% BSA-0.05% Tween 20-0.05% proclin-300) and analysed in a microtitration plate that was coated with the peptide antigen and blocked with BSA-PBS. Alkaline phosphatase-coupled rabbit antichicken/turkey antibodies as a secondary antibody and paranitrophenylphosphate as a substrate, were used. The unsupplemented marmalade samples used as blanks gave negative results (Abs<0.15).

Results of examples 1 and 2 (Elisa-method) are presented in the Figure 1. The control shows the situation, where 25 mg of the antibody was added to the 10 ml of physiological saline, and 50 ug/l of this sample was taken and analysed in a way described in the example 1. The results show that lowering pH decreases the biological binding of the antibody. In a similar way the regulation of pH (buffering) preserves the binding of the antibody. When the antibody was casted in agarose (cloud berry marmalade), high sugar content may have helped restoration of the biological activity of the antibody in low pH. The results indicate that the antibodies added to the marmalade dissolved and retained in mouth where they could be sampled and analysed for the binding to their original antigen.

## Claims

1. A method producing jelly sweets, **characterized in that** antibodies are added to the gel-like or jellyish sweets or marmalade at low temperature before the sweets are allowed to cool without any raising of temperature afterwards.

2. A method according to claim 1, **characterized in that** the antibodies are added at temperatures around 40 °C.

3. A method according to claim 1 or 2, **characterized in that** the antibodies are added to the sweets after the solution is poured into the molds.

4. A method according to claim 3, **characterized in that** the antibodies are added to the sweets before the sweets are solidified.

5. A method according to claim 3, **characterized in that** the antibodies are added to the sweets, into cavities inside the sweets, after the sweets have solidified.

6. A method according to any of claims 1-5, **characterized in that** the antibodies are derived from colostrum or milk.

7. A method according to any of claims 1-5, **characterized in that** the antibodies are derived from egg yolk of hen or other birds.

8. A method according to any of claims 1-7, **characterized in that** the antibodies are filtrated for sterility or purification.

9. A method according to claims 7 and 8, **characterized in that** the antibodies are first extracted from egg yolk to a solution and sterile filtrated before addition.

10. A method according to any of the claims 1- 9, **characterized in that** the pH of the jelly sweets is adjusted.

11. A method according to any of the claims 1-10, **characterized in that** the antibodies inside the jelly sheets are preserved using buffered solutions, trehaloses or other sugars or other corresponding molecules in helping the preservation of antibodies.

## Patentansprüche

1. Verfahren zur Herstellung von Geleesüßwaren, **dadurch gekennzeichnet, dass** den gel- oder geleeartigen Süßwaren oder Marmeladen Antikörper bei niedriger Temperatur zugesetzt werden, ehe man die Süßwaren ohne jegliche darauf folgende Temperaturerhöhung abkühlen lässt.

2. Verfahren nach Anspruch 1, **dadurch gekennzeichnet, dass** die Antikörper bei Temperaturen von etwa 40°C zugesetzt werden.

3. Verfahren nach Anspruch 1 oder 2, **dadurch gekennzeichnet, dass** die Antikörper den Süßwaren zugesetzt werden, nachdem die Lösung in die Formen gegossen wurde.

4. Verfahren nach Anspruch 3, **dadurch gekennzeichnet, dass** die Antikörper den Süßwaren zugesetzt werden, ehe die Süßwaren fest geworden sind.

5. Verfahren nach Anspruch 3, **dadurch gekennzeichnet, dass** die Antikörper den Süßwaren in Hohlräume innerhalb der Süßwaren zugesetzt werden, nachdem die Süßwaren fest geworden sind.

6. Verfahren nach einem beliebigen der Ansprüche 1-5, **dadurch gekennzeichnet, dass** die Antikörper aus Kolostrum oder Milch gewonnen werden.

7. Verfahren nach einem beliebigen der Ansprüche 1-5, **dadurch gekennzeichnet, dass** die Antikörper aus dem Dotter von Eiern von Hühnern oder anderen Vogelarten gewonnen werden.

8. Verfahren nach einem beliebigen der Ansprüche 1-7, **dadurch gekennzeichnet, dass** die Antikörper zur Sterilisation oder Reinigung filtriert werden.

9. Verfahren nach Anspruch 7 und 8, **dadurch gekennzeichnet, dass** die Antikörper vor dem Zusetzen zunächst aus Eidotter in eine Lösung extrahiert und sterilfiltriert werden.

10. Verfahren nach einem beliebigen der Ansprüche 1-9, **dadurch gekennzeichnet, dass** der pH-Wert der Geleesüßwaren eingestellt wird.

11. Verfahren nach einem beliebigen der Ansprüche 1-10, **dadurch gekennzeichnet, dass** die Antikörper in den Geleesüßwaren mit Pufferlösungen, Trehalosen oder anderen Zuckern oder anderen entsprechenden Molekülen, die zum Konservieren von Antikörpern beitragen, konserviert werden.

## Revendications

1. Procédé de production de bonbons gélifiés, **caractérisé en ce que** des anticorps sont ajoutés aux bonbons ou à la confiture de type gelée ou gélifiés à basse température avant de laisser les bonbons refroidir sans augmentation de température consécutive.

2. Procédé selon la revendication 1, **caractérisé en ce que** les anticorps sont ajoutés à des températures avoisinant les 40° C.

3. Procédé selon la revendication 1 ou 2, **caractérisé en ce que** les anticorps sont ajoutés aux bonbons après que la solution a été versée dans les moules.

4. Procédé selon la revendication 3, **caractérisé en ce que** les anticorps sont ajoutés aux bonbons avant la solidification des bonbons.

5. Procédé selon la revendication 3, **caractérisé en ce que** les anticorps sont ajoutés aux bonbons dans des cavités situées à l'intérieur des bonbons une fois que les bonbons se sont solidifiés.

6. Procédé selon l'une quelconque des revendications 1 à 5, **caractérisé en ce que** les anticorps sont dérivés de colustrum ou de lait.

7. Procédé selon l'une quelconque des revendications 1 à 5, **caractérisé en ce que** les anticorps sont dérivés de jaune d'oeuf de poule ou d'autres volatiles.

8. Procédé selon l'une quelconque des revendications 1 à 7, **caractérisé en ce que** les anticorps sont filtrés pour les stériliser ou les purifier.

9. Procédé selon l'une quelconque des revendications 7 et 8, **caractérisé en ce que** les anticorps sont d'abord extraits du jaune d'oeuf pour être mis dans une solution et filtrés pour les stériliser avant leur ajout.

10. Procédé selon l'une quelconque des revendications 1 à 9, **caractérisé en ce que** le pH des bonbons gélifiés est ajusté.

11. Procédé selon l'une quelconque des revendications 1 à 10, **caractérisé en ce que** les anticorps mis dans les bonbons sont conservés en utilisant des solutions tampons, des tréhaloses ou des sucres ou d'autres molécules équivalentes pour aider à la préservation des anticorps.
